# EUROPÄISCHE PATENTSCHRIFT

(11) **EP 1 062 972 B1**
(45) Veröffentlichungstag und Bekanntmachung des Hinweises auf die Patenterteilung: **25.05.2011**
(21) Anmeldenummer: 00250204.5
(22) Anmeldetag: 23.06.2000
(51) Int. Cl.: A61N 1/05

(54) **Elektrodenanordnung**
Electrode assembly
Ensemble électrode

(30) Priorität: 25.06.1999 DE 19930237
(43) Veröffentlichungstag der Anmeldung: 27.12.2000
(73) Patentinhaber: BIOTRONIK SE & Co. KG, 12359 Berlin (DE)
(72) Erfinder: Schaldach, Max, Prof. Dr., 91054 Erlangen (DE); Witte, Joachim, Prof. Dr., 10409 Berlin (DE); Poga, Sven, 48163 Münster (DE)
(74) Vertreter: Lindner-Vogt, Karin L.

(56) Entgegenhaltungen:
- EP-A- 0 408 358
- DE-A- 3 020 586
- US-A- 5 383 924
- US-A- 5 876 408

## Beschreibung

Die Erfindung betrifft eine Elektrodenanordnung mit einer Elektrodenleitung, die eine ihr Inneres gegenüber der Umgebung abdichtende geschlossene Hülle und an ihrem distalen Ende als Elektroden dienende elektrisch leitende Oberflächenbereiche sowie Fixationsmittel zur Verankerung des distalen Endes in einem Körpergewebe, insbesondere einem Herzgewebe aufweist, wobei die Fixationsmittel von der übrigen Elektrodenleitung derart trennbar gestaltet sind, daß die übrige Elektrodenleitung an ihrem distalen Ende auch bei abgetrennten Fixationsmittel geschlosssen ist, so daß das Innere der Elektrodenleitung auch bei abgetrennten Fixationselement gegenüber der Umgebung abgedichtet ist.

Derartige Elektrodenanordnungen sind bekannt, beispielsweise aus der DE 30 20 586. Neben den genannten Elektrodenanordnungen mit sogenannten Tines, kleinen widerhakenartigen Finnen am äußersten distalen Ende der Elektrodenleitung, sind auch Elektrodenleitungen mit Einschraubspitzen bekannt. Die beschriebenen Fixationsmittel dienen dazu, das distale Ende der Elektrodenleitung im Herzgewebe zu verankern, um es so in einer definierten Position zu halten und möglichst gleichbleibende Stimulationsbedingungen aufrecht zu erhalten.

Eine Elektrodenleitung wird beispielsweise über eine Vene in ein Atrium oder ein Ventrikel eines menschlichen Herzens eingeführt. In einigen Fällen muß eine Elektrodenleitung wieder entfernt werden. Dies ist in solchen Fällen problematisch, in denen die Elektrodenleitung mittels der beschriebenen Fixationsmittel im Herzgewebe, dem Myokard, verankert ist, da die Fixationsmittel mit der Zeit in das Gewebe einwachsen. Dies erschwert das Entfernen der Herzschrittmacherelektroden beispielsweise zum Zwecke des Austausches.

Daher wurden Elektrodenleitungen mit abtrennbaren Fixationsmitteln vorgeschlagen, wie sie aus den US-Patenten 5,383,924 und 5,908,447 sowie der bereits genannten DE 30 20 586 hervorgehen.

Eine Indikation für das Entfernen einer Elektrodenleitung ist dann gegeben, wenn sich im Bereich eines mit der Elektrodenleitung verbundenen Schrittmachers oder der Elektrodenleitung selbst Infektionen ergeben haben. In solchen Fällen soll das Entfernen der Elektrodenleitung möglichst nicht zum Ausbreiten der Infektion beitragen. Die bekannten Elektrodenleitungen lassen in dieser Hinsicht zu wünschen übrig.

Weiterhin wachsen nach einigen Jahren nicht nur die Fixationsmittel in das Myokard ein, sondern auch die Elektrodenleitung selbst wird beispielsweise da, wo sie an einer Gefäßwand, beispielsweise der Vena cava, anliegt, von Gewebe eingeschlossen. Das Entfernen der Elektrodenleitung soll in solchen Fällen mit möglichst geringen Verletzungen des einschließenden Gewebes einhergehen.

Ziel der Erfindung ist es, eine Elektrodenanordnung anzugeben, die einfach und ohne unnötige Gesundheitsrisiken zu entfernen ist.

Erfindungsgemäß wird dieses Ziel mit einer Elektrodenanordnung der wie sie im Anspruch 1 definiert ist erreicht, die Mittel zum Abtrennen der Fixationsmittel aufweist, welche ausgebildet sind, ein Abtrennen der Fixationsmittel aufgrund axial innerhalb der Elektrodenleitung wirkender Kräfte zu bewirken.

Der Erfindung liegt der Gedanke zugrunde, solche Belastungen des Patienten zu vermeiden, die durch vermeidbare Bewegungen im Bereich der Außenhülle der Elektrodenleitung entstehen. Solche vermeidbaren Bewegungen sind zum Beispiel das Drehen der Elektrodenleitung aus der DE 30 20 586 um ihre Längsachse, um die Elektrodenleitung von dem dort beschriebenen Fixationsmittel loszuschrauben. Ebenfalls sollen solche Belastungen vermieden werden, die von einer über die Elektrodenleitung zu schiebenden Trennvorrichtung ausgehen, wie sie in dem US-Patent 5,383,924 beschrieben sind. Schließlich sollen die Gesundheitsrisiken vermieden werden, die von einer nach dem Trennen von den Fixationsmitteln am distalen Ende offenen Elektrodenleitung ausgehen, da aus diesem offenen Ende der Elektrodenleitung septische Flüssigkeiten im Inneren der Elektrodenleitung austreten können. Diese Überlegungen haben schließlich zu der Erkenntnis geführt, daß Elektrodenleitungen, die von den Fixationsmitteln allein durch in der Elektrodenleitung wirkende Längskräfte zu trennen sind, alle Anforderungen erfüllen. Dieser Erfindungsgedanke schließt zwei Erfindungsvarianten ein, nämlich eine Variante, bei der ein rein passives Trennen der Fixationsmittel von der übrigen Elektrodenleitung allein durch Ziehen an der Elektrodenleitung erfolgt, und eine andere Variante, bei der die Trennung von Fixationsmittel und Elektrodenleitung durch in der Elektrodenleitung vorgesehene Mittel aktiv bewirkt oder unterstützt wird.

Ein vorteilhaftes Merkmal der erfindungsgemäßen Elektrodenleitung ist, daß diese nach dem Trennen von den Fixationsmitteln über ihre gesamte Länge einen einheitlichen Durchmesser haben, oder sich zum distalen Ende hin verjüngen. Dies erleichtert das Herausziehen der Elektrodenleitungen, falls diese an irgendeiner Stelle von Gewebe eingeschlossen sein sollten.

Erfindungsgemäß sind die Fixationsmittel mit der übrigen Elektrode über eine Solltrennstelle derart verbunden, daß die Verbindung im Bereich der Solltrennstelle durch definierte, durch die Gestaltung der Solltrennstelle vorgebene Kräfte trennbar ist. Die definiert vorgegebenen Kräfte bewirken ein Lösen der Fixationsmittel von der übrigen Elektrodenleitung, vorzugsweise unter Einfluß solcher Kräfte, wie sie vom Herzgewebe als Reaktion auf auf die Elektrode ausgeübte Zugkräfte in Längsrichtung der Elektrodenleitung ausgeübt werden können. Die zum Trennen der Solltrennstelle erforderlichen Kräfte hängen von deren Konstruktion ab. Einflußgrößen sind die Geometrie der Verbindung sowie die Art des verwendeten Materials. Durch Variation dieser Einflußgrößen ist es dem Fachmann möglich, die Solltrennstelle so zu gestalten, daß vom Herzgewebe selbst auszuübende Kräfte ausreichen, um die Fixationsmittel von der übrigen Elektrodenleitung zu trennen. Eine derartige Gestaltung der Solltrennstelle erlaubt es, das Ende der Elektrodenleitung einerseits sicher zu verankern und andererseits die Elektrodenleitung zu Austauschzwecken ohne Verletzung des Herzgewebes zu entfernen.

In einer bevorzugten Ausführungsform besitzen die Fixationsmittel oder die Elektrodenleitung einen Vorsprung im Bereich der Solltrennstelle, der in eine entsprechende Vertiefung im jeweils anderen Teil eingreift. Eine derartige Verrastung der Solltrennstelle kann leicht so gestaltet werden, daß sie sich unter Einfluß definierter Kräfte trennt.

Eine alternative, ebenso bevorzugte Ausführungsform zeichnet sich durch eine lösbare mechanische Kupplung zwischen den Fixationsmitteln und der übrigen Elektrodenleitung aus, sowie durch ein mit der Kupplung in Eingriff zu bringendes Betätigungsmittel zum Lösen der Kupplung. Im Gegensatz zu der vorbeschriebenen Ausführungsvariante, bei der sich die Solltrennstelle passiv in Reaktion auf den Einfluß definierter Kräfte trennt, kann die Verbindung zwischen Fixationsmitteln und der übrigen Elektrodenleitung bei der zuletzt genannten Ausführungsvariante aktiv mit Hilfe des entsprechenden Betätigungsmittels gelöst werden. Der Vorteil der letztgenannten Ausführungsvariante liegt darin, daß die beim Austauschen einer Elektrodenleitung im Myokard wirkenden Kräfte noch geringer sein können. Andererseits ist dafür ein größerer konstruktiver Aufwand der Elektrodenleitung erforderlich.

In einer bevorzugten Ausgestaltung der letztgenannten Ausführungsvariante weist die Kupplung Rastelemente auf, die einen Vorsprung an den Fixationsmitteln hintergreifen und die durch das Betätigungsmittel in eine den Vorsprung freigebende Stellung zu bringen sind. Eine derartige Kupplung kann leicht mit wenigen Bauteilen so realisiertwerden, daßsie zuverlässig arbeitet. Eine detaillierte Beschreibung einer solchen Kupplung findet sich in der Figurenbeschreibung.

Im Falle einer Elektrodenanordnung mit einer als Tipelektrode dienenden elektrisch leitenden Oberfläche an ihrem distalen Ende ist die Solltrennstelle in einer bevorzugten Ausführungsvariante derart gestaltet, daß die Fixationsmittel von der übrigen Elektrodenleitung so trennbar sind, daß die Tipelektrode beim Trennen an der Elektrodenleitung verbleibt.

Eine alternative, ebenso bevorzugte Ausführungsvariante zeichnet sich dadurch aus, daß die Elektrodenleitung elektrische Kontaktelemente zum Kontaktieren der Tipelektrode aufweist, so daß die Fixationsmittel derart von der übrigen Elektrodenleitung trennbar sind, daß die Tipelektrode beim Trennen an den Fixationsmitteln verbleibt.

Die Fixationsmittel weisen bevorzugt Markierungsmittel auf, die durch elektromagnetische oder akustische Ortungsmittel, wie beispielsweise ein Röntgengerät, ortbar sind. Solche Markierungsmittel erlauben es, nach dem Entfernen einer Elektrodenleitung im Myokard verbliebene Fixationsmittel zu orten und eine neueinzuführende Elektrodenleitung so zu plazieren, daß deren Fixationsmittel nicht am gleichen Ort wie die vorhergehenden Elektrodenleitung in das Herzgewebe eindringen.

Die Markierungsmittel enthalten vorzugweise Gold, da Gold körperverträglich und röntgenopak, das heißt auf Röntgenbildern sichtbar ist.

Falls die Fixationsmittel solche widerhakenartig ausgebildeten Tines sind, sind die Markierungsmittel vorzugsweise in den Tines angeordnet.

Erfindungsgemäß ist die Elektrodenanordnung durch eine beim Verbinden der Fixationsmittel mit der übrigen Elektrodenleitung plastische Kunststoffmasse im Bereich der Solltrennstelle gekennzeichnet. Die plastische Kunststoffmasse enthält vorzugsweise Silikon. Die Kunststoffmasse kann wahlweise oder gleichzeitig Dichtungs- und Adhäsions-, also Klebefunktion, übernehmen. Da sie beim Verbinden der Fixationsmittel mit der übrigen Elektrodenleitung flüssig bzw. plastisch ist, ist sie geeignet, alle Spalten, die zwischen den Fixationsmitteln der übrigen Elektrodenleitung verbleiben könnten, vollständig auszufüllen.

Es ist zu beachten, daß sich die Merkmale der Erfindung auch auf solche Elektrodenanordnungen übertragen lassen, die anstelle von Fixationsmitteln mit sogenannten Tines beispielsweise korkenzieherartig ausgebildete Einschraubspitzen als Fixationsmittel und gegebenfalls zusätzlich als Elektrode besitzen. Auch derartige Einschraubspitzen können mit der übrigen Elektrodenleitung in der zuvor beschriebenen wie auch in der Figurenbeschreibung beschriebenen Weise verbunden sein.

Die Erfindung soll nun anhand von Ausführungsbeispielen mit Hilfe der Figuren näher erläutert werden. Die Figuren zeigen:
- Figur 1a bis c: das distale Ende einer Elektrodenleitung mit aktiv abtrenn- baren Fixationsmitteln;
- Figur 2: die Elektrodenleitung aus Figur 1 eingesetzt und verankert im Ventrikel eines menschlichen Herzens;
- Figur 3: eine alternative Ausführungsform eines distalen Endes einer Elek- trodenleitung mit passiv trennbaren Fixationsmitteln und
- Figur 4: eine weitere alternative Ausführungsform des distalen Endes einer Elektrodenleitung mit trennbaren Fixationsmitteln.

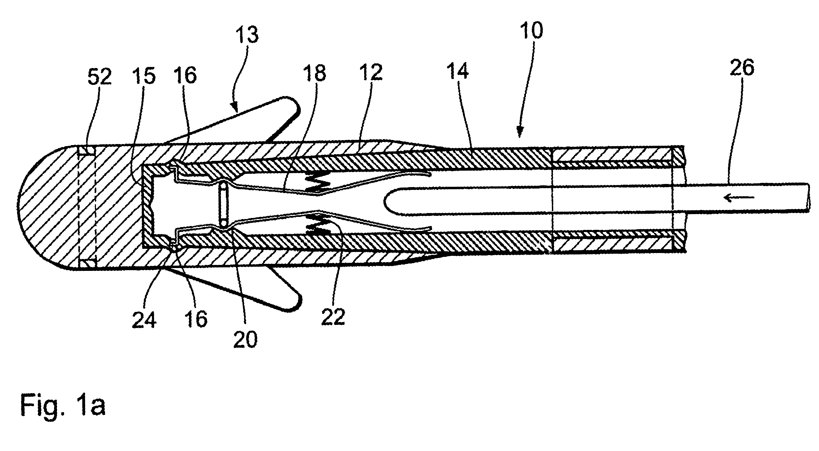

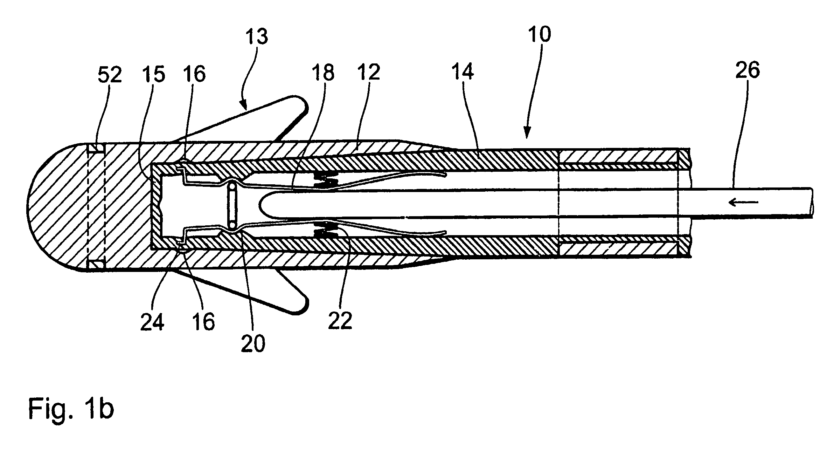

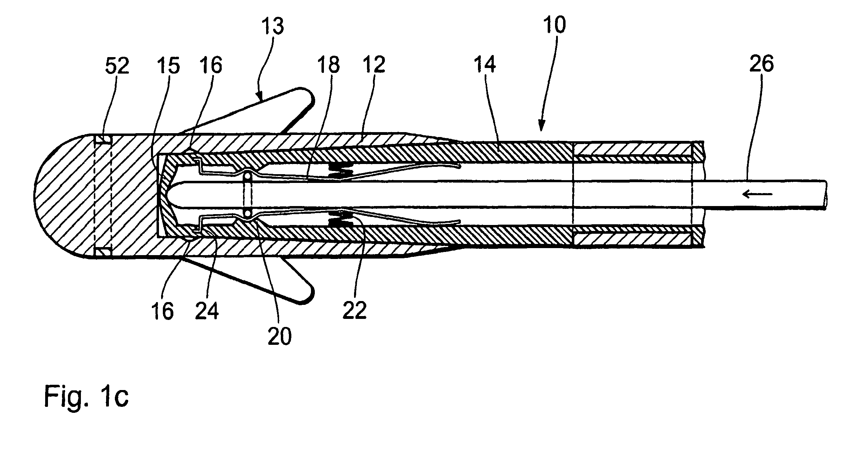

Das in Figur 1 abgebildete distale Ende einer Elektrodenleitung 10 besitzt Fixationsmittel 12 mit widerhakenartigen Tines 13 aus Silikonkunststoff. Die Elektrodenleitung weist eine rundum geschlossene Hülle 14 auf, die das Innere der Elektrodenleitung gegenüber ihrer Umgebung abdichtet. Die Hülle 14 besitzt an ihren distalen Ende eine geschlossene Stirnwand 15. Die Fixationsmittel 12 bilden eine Kappe am Ende der Elektrodenleitung 10 und sind mit deren Hülle 14 über eine mechanische Kupplung verbunden, die auf seiten der Hülle 14 von Vorsprüngen 16 gebildet wird, welche in entsprechende Vertiefungen in dem Fixationsmittel 12 eingreifen. Die Hülle 14 wird im Bereich der Vorsprünge 16 von Federlaschen 18 nach außen gedrückt, die bei 20 schwenkbar gelagert sind. Druckfedern 22 unterstützen den an ihren distalen Enden 24 nach außen gerichteten Druck der Federlaschen 18, sind aufgrund der Formgebung der Federlaschen 18 aber nicht unbedingt erforderlich. Die Funktion der Druckfedern 22 kann auch alleine von den integral an die Federlaschen 18 angeformten Blattfedern übernommen werden. An ihren distalen Enden 24 sind die Federlaschen 18 mit der Hülle 14 verbunden, und zwar im Bereich der Vorsprünge 16.

Zum Lösen der mechanischen Kupplung dient ein Mandrin 26, der durch Längsverschieben der durch den Pfeil in Figur 1 angedeuteten Richtung zwischen die Federlaschen 18 gerät und diese gegen die Federspannung an ihren dem Mandrin nahen Ende auseinanderdrückt, so daß die abgebogenen rastenden Enden 24 der Federlaschen 18 die Vorsprünge 16 in der Hülle 14 nach innen aus den entsprechenden Vertiefungen in den Fixationsmitteln 12 zurückziehen und diese freigeben; siehe Figur 1b.

Durch weiteres Einschieben des Mandrin 26 in der bezeichneten Richtung erreicht dieser die Stirnwand 15 und beult diese nach außen aus; siehe Figur 1c. Dadurch werden die Fixationsmittel 12 bezüglich der Elektrodenleitung 10 nach vorne geschoben und leicht gelöst. Da sich die Hülle 14 dort, wo sie von den Fixationsmitteln 12 umgeben ist, zu ihrem distalen Ende hin leicht verjüngt, ergibt sich sofort eine Trennung von Elektrodenleitung 10 und Fixationsmitteln 12. Das Verjüngen der Hülle 14 wird durch das Ausbeulen der Stirnwand 15 durch den Mandrin 16 noch verstärkt. Auf diese Weise können die Fixationsmittel 12 aktiv von der Elektrodenleitung 10 getrennt werden.

Eine nicht dargestellte Variante der in Figur 1 dargestellten Ausführung weist keine Vorsprünge an der Hülle 14 auf und besitzt auch keine Federlaschen. Die Fixationsmittel werden allein dadurch auf der ansonsten der Hülle 14 entsprechenden Hülle gehalten, daß diese Hülle sich kurz vor ihrem distalen Ende nicht weiter verjüngt oder zum distalen Ende hin im Durchmesser sogar wieder weitet. Ein Mandrin wie der Mandrin 26 dient dann dazu, die Stirnwand 15 wie beschrieben auszubeulen und damit gleichzeitig eine Verjüngung des distalen Endes der Hülle 14 zu bewirken, welche die auf das distale Ende der Hülle aufgeklemmten Fixationsmittel freigibt und so die Verbindung zwischen Hülle und Fixationsmitteln gelöst.

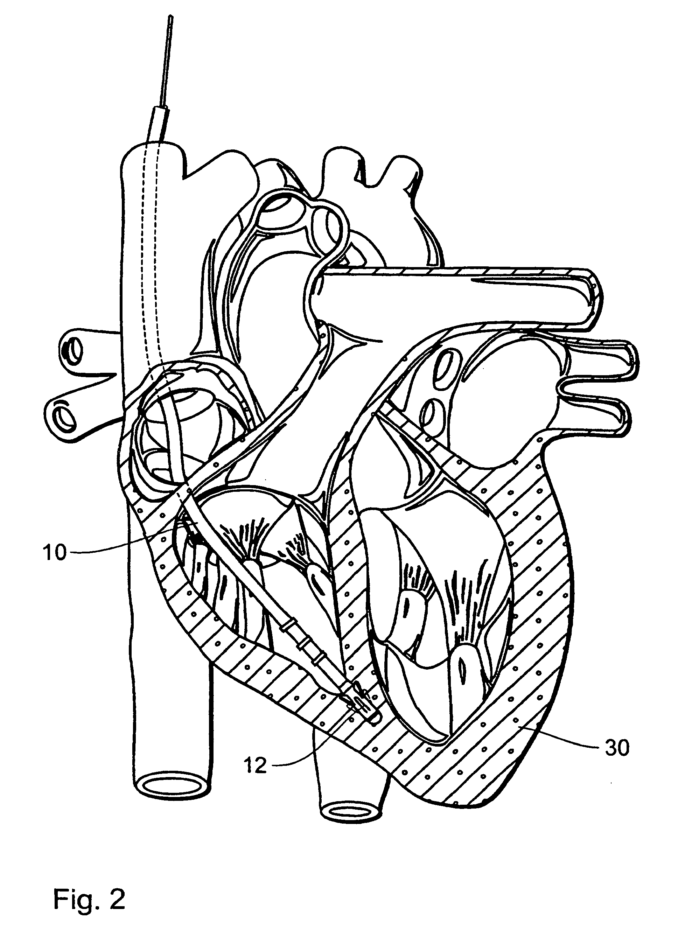

Figur 2 zeigt die Elektrodenleitung 10 aus Figur 1 mit deren Fixationsmitteln 12 im Herzgewebe oder Myokard eines Ventrikels eines menschlichen Herzens 30 verankert. Die Fixationsmittel 12 wachsen mit der Zeit in das Myokard des Herzens 30 ein, so daß die Elektrodenleitung 10 nicht ohne Verletzung des Myokards entfernt werden kann. Um die Elektrodenleitung 10 zu entfernen, wird die Kupplung zwischen den Fixationsmitteln 12 und der Elektrodenleitung 10 auf die zuvor beschriebene Weise gelöst, so daß die Elektrodenleitung 10 ohne Belastung des Myokards entfernt werden kann und die Fixationsmittel 12 im Myokard verbleiben. Die Fixationsmittel 12 können beispielsweise vollständig aus Silikonkunststoff bestehen und sind für das Herz unschädlich.

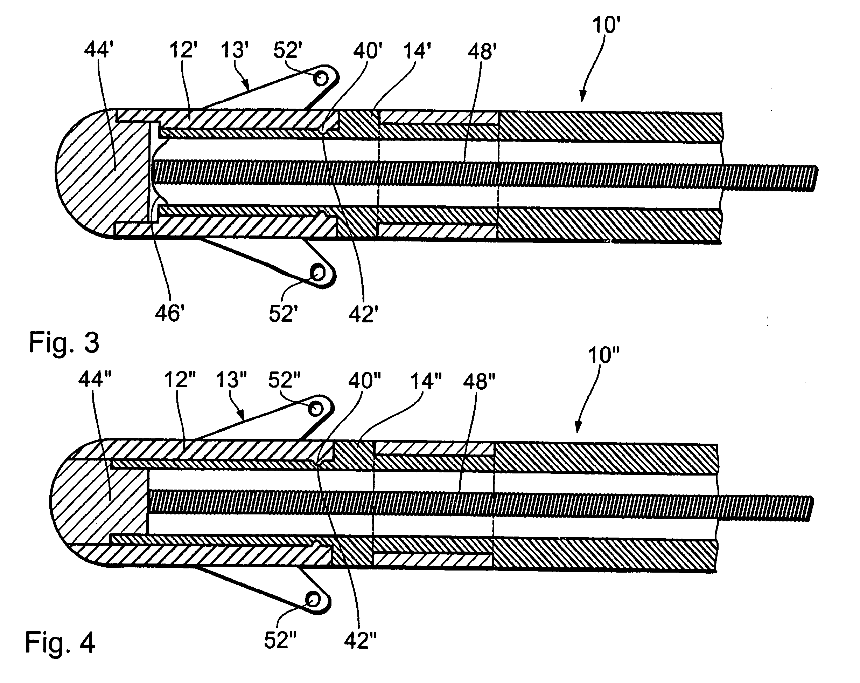

Figur 3 zeigt eine alternative Ausführungsform einer Elektrodenleitung 10' mit Fixationsmitteln 12' an deren distalem Ende. Die Fixationsmittel 12' sind mit der übrigen Elektrodenleitung 10' verrastet. Dazu dient eine Lippe 40' an den Fixationsmitteln, die in eine Vertiefung 42' in der Hülle 14' der Elektrodenleitung 10' eingreift. Mit den Fixationsmitteln 12' ist eine Tipelelektrode 44' verbunden, die dem Abgeben von elektrischen Spannungsimpulsen direkt an das Myokard oder umgekehrt zum Aufnehmen elektrischer Signale aus dem Myokard dient. Die Tipelektrode 44' besteht aus Metall und wird von einem Kontakt 46' kontaktiert, der über eine gewendelte elektrische Leitung 48' mit einer Vorrichtung zum Abgeben elektrischer Impulse wie beispielsweise einem Defibrillator oder einem Herzschrittmacher in Verbindung steht. Der Kontakt 46' dient gleichzeitig als das distale Ende der Elektrodenleitung 10' abdichtende Stirnwand der Hülle 14'. Der Kontakt 46' ist dazu mit seinem gesamten Umfang in die Hülle 14' eingelassen.

Die Verrastung der Fixationsmittel 12' mit der übrigen Elektrodenleitung 10' mittels der Lippe 40' und der Grube 42' ist so gestaltet, daß vom Myokard auf die Fixationsmittel 12' auszuübende Kräfte ausreichen, um die Verbindung zwischen den Fixationsmitteln 12' und der übrigen Elektrodenleitung 10' zu trennen. Dies hat zur Folge, daß sich die Fixationsmittel 12' von der übrigen Elektrodenleitung 10' lösen, wenn die Elektrodenleitung 10' aus dem Ventrikel herausgezogen wird. Die Fixationsmittel 12' verbleiben zusammen mit der Tipelektrode 44' im Myokard des Herzens 30.

Wie Figur 4 zeigt, können eine Elektrodenleitung 10" und Fixationsmittel 12'' auch so gestaltet sein, daß eine Tipelektrode 44'' fester Bestandteil der Elektrodenleitung 10'' ist, und am distalen Ende der Elektrodenleitung 10'' verbleibt, wenn die Fixationsmittel 12'' von der übrigen Elektrodenleitung 10" getrennt werden.

Weitere, bisher nicht erwähnte Merkmale der Elektrodenleitungen 10, 10' und 10'' aus den Figuren 1, 3 und 4 sind unter anderem Ringelektroden 50, die auf der Elektrodenleitung 10, 10' und 10'' zur Abgabe elektrischer Impulse oder zur Aufnahme elektrischer Signale angeordnet sind. Außerdem können im Bereich der Verbindung der Elektrodenleitung 10, 10' und 10'' mit den Fixationsmitteln 12, 12' und 12'' zunächst plastische Silikonkunststoffe vorgesehen sein, die nach Art einer Dichtmasse verbliebene Spalten dichten können und zusätzlich durch Adhäsionskräfte zu der Elektrodenleitung bzw. den Fixationsmitteln Einfluß auf die Kraft haben, mit der die Fixationsmittel an der Elektrodenleitung befestigt sind.

Sämtliche Fixationsmittel 12, 12' und 12'' weisen Markierungsmittel auf, die im Falle des Fixationsmittels 12 aus Fig. 1 als Ring ausgebildet und in den Figuren 3 und 4 an den Tines befestigt sind. Die Markierungsmittel 52 enthalten Gold, das körperverträglich und röntgenopak, d.h. mittels eines Röntgengerätes sichtbar ist. Auf diese Weise können die markierten Fixationsmittel 12, 12' und 12'' beim Einsetzen einer neuen Elektrodenleitung mittels eines Röntgengerätes geortet werden.

## Patentansprüche

1. Elektrodenanordnung mit einer Elektrodenleitung (10, 10', 10''), die eine ihre Inneres gegenüber der Umgebung abdichtende geschlossene Hülle (14, 14', 14'') und an ihrem distalen Ende als Elektroden dienende elektrisch leitende Oberflächenbereiche (50) sowie Fixationsmittel (12, 12', 12'') zur Verankerung des distalen Endes in einem Körpergewebe, insbesondere einem Herzgewebe aufweist, wobei die Fixationsmittel (12, 12', 12'') von der übrigen Elektrodenleitung (10, 10', 10'') derart trennbar gestaltet sind, daß die übrige Elektrodenleitung an ihrem distalen Ende auch bei abgetrennten Fixationsmittel geschlosssen ist, so dass das Innere der Elektrodenleitung auch bei abgetrennten Fixationselement gegenüber der Umgebung abgedichtet ist, und Mittel zum Abtrennen der Fixationsmittel (12, 12', 12"), vorgesehen sind, die ausgebildet sind, ein Abtrennen der Fixationsmittel (12, 12', 12'') aufgrund axial innerhalb der Elektrodenleitung (10, 10', 10") wirkender Kräfte zu bewirken, wobei die
Fixationsmittel (12', 12'') mit der übrigen Elektrodenleitung (10', 10'') über eine Solltrennstelle derart verbunden sind, daß die Verbindung im Bereich der Solltrennstelle durch definierte, durch die Gestaltung der Solltrennstelle vorgegebene Kräfte trennbar ist, **gekennzeichnet durch** plastische Kunststoffmasse im Bereich der Solltrennstelle.

2. Elektrodenanordnung nach Anspruch 1, **dadurch gekennzeichnet, daß** die definiert vorgegebenen Kräfte ein Lösen der Fixationsmittel (12', 12'') von der übrigen Elektrodenleitung (10', 10'') unter dem Einfluß solcher Kräfte bewirken, wie sie vom Herzgewebe als Reaktion auf auf die Elektrodenleitung (10', 10") ausgeübte Zugkräfte in Längsrichtung der Elektrodenleitung ausübbar sind.

3. Elektrodenanordnung nach Anspruch 1 oder 2, **dadurch gekennzeichnet, daß** die Fixationsmittel (12', 12'') oder die Elektrodenleitung (10', 10'') einen Vorsprung (40', 40'') im Bereich der Solltrennstelle aufweist, der in eine entsprechende Vertiefung (42', 42'') im jeweils anderen Teil eingreift.

4. Elektrodenanordnung nach Anspruch 1, **gekennzeichnet durch** eine lösbare mechanische Kupplung zwischen den Fixationsmitteln (12) und der übrigen Elektrodenleitung (10), sowie **durch** ein mit der Kupplung in Eingriff zu bringendes Betätigungsmittel (26) zum Lösen der Kupplung.

5. Elektrodenanordnung nach Anspruch 4, **dadurch gekennzeichnet, daß** die Kupplung wenigstens einen in die Fixationsmitteln (12) eingreifenden Vorsprung (16) aufweist, der durch das Betätigungsmittel (26) in eine die Fixationsmittel (12) freigebende Stellung bringbar ist.

6. Elektrodenanordnung nach einem der Ansprüche 1 bis 5, mit einem als Tipelektrode (44') dienenden elektrisch leitenden Oberflächenbereich am distalen Ende der Elektrodenleitung (10'), **dadurch gekennzeichnet, daß** die Fixationsmittel (12') derart von der übrigen Elektrodenleitung (10') trennbar sind, daß die Tipelektrode (44') beim Trennen an der Elektrodenleitung verbleibt.

7. Elektrodenanordnung nach einem der Ansprüche 1 bis 5, mit einem als Tipelektrode (44'') dienenden elektrisch leitenden Oberflächenbereich am distalen Ende der Elektrodenleitung (10''), **dadurch gekennzeichnet, daß** die Elektrodenleitung (10'') elektrische Kontaktelemente zum Kontaktieren der Tipelektrode (44'') aufweist und daß die Fixationsmittel (12'') derart von der übrigen Elektrodenleitung (10'') trennbar sind, daß die Tipelektrode (44'') beim Trennen an den Fixationsmitteln (12'') verbleibt.

8. Elektrodenanordnung nach einem der Ansprüche 1 bis 7, **dadurch gekennzeichnet, daß** die Fixationsmittel (12, 12', 12'') Markierungsmittel (52) aufweisen, die durch elektromagnetische oder akustische Ortungsmittel wie ein Röntgengerät ortbar sind.

9. Elektrodenanordnung nach Anspruch 8, **dadurch gekennzeichnet, daß** die Markierungsmittel (52) Gold enthalten.

10. Elektrodenanordnung nach Anspruch 8 oder 9, mit Fixationsmitteln, die widerhakenartig ausgebildete Tines (14, 14', 14'') besitzen, **dadurch gekennzeichnet, daß** die Markierungsmittel (52) in den Tines (14, 14', 14'') angeordnet sind.

11. Elektrodenanordnung nach Anspruch 1, **dadurch gekennzeichnet, daß** die Kunststoffmasse Silikon enthält.

## Claims

1. An electrode arrangement comprising an electrode lead (10, 10', 10") which has a sleeve (14, 14', 14"), which is closed and seals the interior thereof against the surroundings, and electrically conductive surface regions (50) on the distal end thereof which function as electrodes, and fixation means (12, 12', 12") for anchoring the distal end in bodily tissue, in particular cardiac tissue, wherein the fixation means (12, 12', 12") are designed to be separable from the rest of the electrode lead (10, 10', 10") in a manner such that the rest of the electrode lead is closed on the distal end thereof even if the fixation means have been removed, thereby ensuring that the interior of the electrode lead is sealed against the surroundings even if the fixation element has been removed, and means are provided for removing the fixation means (12, 12', 12"), which are designed such that the fixation means (12, 12', 12") are removed using forces that act axially inside the electrode lead (10, 10', 10"), wherein the fixation means (12', 12") are connected to the rest of the electrode lead (10', 10") via a predetermined breaking point in a manner such that the connection can be disconnected in the region of the predetermined breaking point using forces determined by the design of the predetermined breaking point, **characterized by** a mass of plastic polymer material in the region of the predetermined breaking point.

2. The electrode arrangement according to claim 1, **characterized in that** the predefined forces cause the fixation means (12', 12") to become detached from the rest of the electrode lead (10', 10") under the influence of forces such as those that can be exerted by cardiac tissue in response to tension forces applied to the electrode lead (10', 10") in the longitudinal direction of the electrode lead.

3. The electrode arrangement according to claim 1 or 2, **characterized in that** the fixation means (12', 12") or the electrode lead (10', 10") comprises a projection (40', 40") in the region of the predetermined breaking point, which engages in a corresponding recess (42', 42") in the respective other part.

4. The electrode arrangement according to claim 1, **characterized by** a detachable mechanical connection between the fixation means (12) and the rest of the electrode lead (10), and by an actuating element (26) which is to be engaged with the coupling to detach the coupling.

5. The electrode arrangement according to claim 4, **characterized in that** the coupling comprises at least one projection (16) which engages in the fixation means (12) and can be moved into a position-using the actuating element (26)-in which the fixation means (12) are released.

6. The electrode arrangement according to one of the claims 1 to 5, comprising an electrically conductive surface region on the distal end of the electrode lead (10'), which is used as a tip electrode (44'), **characterized in that** the fixation means (12') can be disconnected from the rest of the electrode lead (10') in a manner such that the tip electrode (44') remains on the electrode lead after disconnection.

7. The electrode arrangement according to one of the claims 1 to 5, comprising an electrically conductive surface region on the distal end of the electrode lead (10"), which is used as a tip electrode (44"), **characterized in that** the electrode lead (10") comprises electrical contact elements for contacting the tip electrode (44"), and the fixation means (12") can be disconnected from the rest of the electrode lead (10") in a manner such that the tip electrode (44") remains on the fixation means (12") after disconnection.

8. The electrode arrangement according to one of the claims 1 to 7, **characterized in that** the fixation means (12, 12', 12") comprising marking means (52) which can be located using electromagnetic or acoustic locating means such as an X-ray device.

9. The electrode arrangement according to claim 8, **characterized in that** the marking means (52) contain gold.

10. The electrode arrangement according to claim 8 or 9, comprising fixation means which have barbed tines (14, 14', 14"), **characterized in that** the marking means (52) are disposed in the tines (14, 14', 14").

11. The electrode arrangement according to claim 1, **characterized in that** the mass of polymer material contains silicone.

## Revendications

1. Ensemble d'électrodes avec un câble d'électrodes (10, 10', 10"), comportant une gaine fermée (14, 14', 14") isolant son intérieur par rapport à l'environnement, et, à son extrémité distale, des zones de surface (50) électriquement conductrices, servant d'électrodes, ainsi que des moyens de fixation (12, 12', 12") pour l'ancrage de l'extrémité distale dans un tissu corporel, en particulier un tissu cardiaque, dans lequel les moyens de fixation (12, 12', 12") peuvent être séparés de telle manière du reste du câble d'électrodes (10, 10', 10"), que le reste du câble d'électrodes est également fermé à son extrémité distale par des moyens de fixation détachés, de sorte que l'intérieur du câble d'électrodes est isolé par rapport à l'environnement même lorsque l'élément de fixation est séparé, dans lequel sont prévus des moyens pour la séparation des moyens de fixation (12, 12', 12"), conçus pour provoquer une séparation des moyens de fixation (12, 12', 12") à l'aide de forces agissant axialement à l'intérieur du câble d'électrodes (10, 10', 10"), dans lequel les moyens de fixation (12', 12") sont reliés de telle manière avec le reste du câble d'électrodes (10', 10") par un point de séparation théorique, que la liaison peut être défaite dans la région du point de séparation théorique, par des forces prédéfinies par la conception du point de séparation,
**caractérisé par** une masse de matière plastique dans la région du point de séparation.

2. Ensemble d'électrodes selon la revendication 1, **caractérisé en ce que** les forces prédéfinies provoquent un détachement des moyens de fixation (12', 12") par rapport au reste du câble d'électrodes (10', 10"), sous l'influence de forces telles qu'elles peuvent être exercées par le tissu cardiaque, en réaction aux forces de traction exercées sur le câble d'électrodes (10', 10"), dans le sens longitudinal du câble d'électrodes.

3. Ensemble d'électrodes selon la revendication 1 ou 2, **caractérisé en ce que** les moyens de fixation (12', 12") ou le câble d'électrodes (10', 10") comportent une saillie (40', 40") dans la région du point de séparation théorique, qui s'introduit dans un renfoncement correspondant (42', 42") dans l'autre des pièces.

4. Ensemble d'électrodes selon la revendication 1, **caractérisé par** un accouplement mécanique séparable entre les moyens de fixation (12) et le reste du câble d'électrodes (10), ainsi que par un moyen d'actionnement (26) à engager avec l'accouplement, pour la rupture de l'accouplement.

5. Ensemble d'électrodes selon la revendication 4, **caractérisé en ce que** l'accouplement comporte une saillie (16) engagée dans les moyens de fixation (12), qui peut être mise dans la position de libération des moyens de fixation (12) par le moyen d'actionnement (26).

6. Ensemble d'électrodes selon l'une des revendications 1 à 5, avec une zone de surface électriquement conductrice, servant d'électrode à pointe (44'), à l'extrémité distale du câble d'électrodes (10'), **caractérisé en ce que** les moyens de fixation (12') sont séparables de telle manière du reste du câble d'électrodes (10'), que l'électrode à pointe (44') reste sur le câble d'électrodes lors de la séparation.

7. Ensemble d'électrodes selon l'une des revendications 1 à 5, avec une zone de surface électriquement conductrice, servant d'électrode à pointe (44") à l'extrémité distale du câble d'électrodes (10"), **caractérisé en ce que** le câble d'électrodes (10") comporte des éléments de contact électrique, pour la mise en contact de l'électrode à pointe (44"), et **en ce que** les moyens de fixation (12") peuvent être séparés de telle manière du reste du câble d'électrodes (10"), que l'électrode à pointe (44") reste sur les moyens de fixation (12") lors de la séparation.

8. Ensemble d'électrodes selon l'une des revendications 1 à 7, **caractérisé en ce que** les moyens de fixation (12, 12', 12") comportent des moyens de marquage (52), localisables par des moyens de localisation électromagnétiques ou acoustiques, tels qu'un dispositif de radiographie.

9. Ensemble d'électrodes selon la revendication 8, **caractérisé en ce que** les moyens de marquage (52) contiennent de l'or.

10. Ensemble d'électrodes selon la revendication 8 ou 9, avec des moyens de fixation possédant des dents (14, 14', 14") du genre barbillons, **caractérisé en ce que** les moyens de marquage (52) sont installés dans les dents (14, 14', 14").

11. Ensemble d'électrodes selon la revendication 1, **caractérisé en ce que** la masse de matière plastique contient de la silicone.
